# EUROPEAN PATENT APPLICATION

(11) **EP 4 338 783 A1**
(43) Date of publication of application: **20.03.2024**
(21) Application number: 21840769.0
(22) Date of filing: 25.10.2021
(51) Int. Cl.: A61M 37/00, A61K 9/00, B29C 41/12, B29C 41/22, B29C 41/00

(54) **MICRONEEDLE PATCH, METHOD OF MANUFACTURING MICRONEEDLE PATCH, AND APPARATUS FOR MANUFACTURING MICRONEEDLE PATCH**

(30) Priority: 12.05.2021 KR 20210061417; 12.05.2021 KR 20210061451
(71) Applicant: Feroka Inc., Seoul 04784 (KR)
(72) Inventor: LEE, Jae Joon, Seoul 02793 (KR); JEON, Yi Seul, Seoul 08799 (KR)
(74) Representative: Hoffmann Eitle
(86) International application number: PCT/KR2021/015027
(87) International publication number: WO 2022/239915

(57) **Abstract**

Provided are a microneedle patch, a method of manufacturing a microneedle patch, and an apparatus for manufacturing a microneedle patch. The method includes: filling a mold having a plurality of needle grooves with a base material; creating a subatmospheric pressure in the mold; rotating the mold about a rotation axis; and drying the base material filled in the needle grooves.

## Description

### TECHNICAL FIELD

The present disclosure relates to a microneedle patch, a method of manufacturing the microneedle patch, and an apparatus for manufacturing the microneedle patch.

### BACKGROUND ART

Injection of a drug into a human body has traditionally been performed by using a needle syringe, but such needle-syringe injection causes great pain. Non-invasive drug injection methods have been developed to overcome this issue, but these methods have a problem in that a large amount of drug is consumed compared to the amount of actually delivered drug.

In order to find a solution to this problem, many studies have been conducted on drug delivery systems (DDSs), and these studies have made even greater advances with the development of nanotechnology.

Unlike conventional injection needles, microneedles enable painless skin penetration without injury. In addition, a certain degree of physical hardness of microneedles may be required to penetrate the stratum corneum of skin. In addition, an appropriate length of microneedles may be required for physiologically active substances to reach the epidermal or dermal layer of skin. Furthermore, in order to effectively deliver physiologically active substances in hundreds of microneedles into skin, the microneedles need to have high skin permeability and be maintained for a certain period of time until dissolution after being inserted into the skin.

Accordingly, interest in microneedles capable of delivering a precise amount of a drug and accurately setting a target position is increasing.

### DESCRIPTION OF EMBODIMENTS

### TECHNICAL PROBLEM

The present disclosure may provide a microneedle patch capable of effectively delivering a preset amount of an effective ingredient to a target position, and a method and apparatus for manufacturing the microneedle patch.

### SOLUTION TO PROBLEM

An aspect of the present disclosure provides a method of manufacturing a microneedle patch including: filling a mold having a plurality of needle grooves with a base material; creating a subatmospheric pressure in the mold; rotating the mold about a rotation axis; and drying the base material filled in the needle grooves.

Another aspect of the present disclosure provides a method of manufacturing a microneedle patch including: filling a mold having a plurality of needle grooves with a buffer solution; arranging a base material on the needle grooves; diffusing the base material into the buffer solution; and drying the mold.

### ADVANTAGEOUS EFFECTS OF PRESENT DISCLOSURE

By using an apparatus for manufacturing a microneedle patch and a method of manufacturing a microneedle patch according to the present disclosure, a high-quality microneedle patch may be manufactured. According to the present disclosure, a subatmospheric pressure is created in a mold to remove gas remaining in the microneedle patch and remove gas between the mold and a base material, so as to manufacture the microneedle patch that does not include foreign substances in its microneedles, is easy to be attached to the skin of a patient, and effectively delivers a drug.

By using the apparatus for manufacturing a microneedle patch and the method of manufacturing a microneedle patch according to the present disclosure, a high-quality microneedle patch may be manufactured. A vacuum is created in the mold to remove gas remaining in the microneedle patch and remove gas between the mold and the base material, so as to manufacture the microneedle patch that does not include foreign substances in its microneedles, is easy to be attached to the skin of a patient, and effectively delivers a drug.

### BRIEF DESCRIPTION OF DRAWINGS

FIG. 1 is a diagram illustrating an apparatus for manufacturing a microneedle patch, according to an embodiment of the present disclosure.
FIG. 2 is a perspective view of a microneedle patch manufactured by the apparatus for manufacturing a microneedle patch of FIG. 1.
FIG. 3 is a diagram illustrating a base material being injected from an injection module of FIG. 1.
FIG. 4 is a diagram illustrating driving of a pressure module of FIG. 1.
FIG. 5 is an enlarged view of region A of FIG. 4.
FIG. 6 is a diagram illustrating driving of a rotation module.
FIG. 7 is a diagram illustrating filling of a mold with a base material in region B of FIG. 6.
FIG. 8 is a flowchart of a method of manufacturing a microneedle patch, according to another embodiment of the present disclosure.
FIG. 9 is a flowchart of a method of manufacturing a microneedle patch, according to another embodiment of the present disclosure.
FIGS. 10 to12 are diagrams illustrating microneedle patches manufactured by using a method of manufacturing a microneedle patch of the present disclosure.
FIGS. 13 and 14 are diagrams showing microneedle patches manufactured by a method of manufacturing a microneedle patch of the present disclosure and comparative examples.
FIG. 15 is a diagram illustrating an apparatus for manufacturing a microneedle patch, according to another embodiment of the present disclosure.
FIG. 16 is a flowchart of a method of manufacturing a microneedle patch according to another embodiment of the present disclosure.
FIGS. 17 to 21 are diagrams illustrating operations of the method of manufacturing a microneedle patch of FIG. 16.
FIG. 22 is a diagram illustrating a microneedle patch manufactured by the method of manufacturing a microneedle patch of FIG. 16.
FIGS. 23 to 25 are diagrams illustrating other embodiments of the microneedle patch of FIG. 22.

### BEST MODE

An aspect of the present disclosure provides a method of manufacturing a microneedle patch including: filling a mold having a plurality of needle grooves with a base material; creating a subatmospheric pressure in the mold; rotating the mold about a rotation axis; and drying the base material filled in the needle grooves.

In the forming of the subatmospheric pressure in the mold, bubbles in the base material filled in the needle grooves may be removed or gas between the base material and the needle grooves may be removed.

In the rotating of the mold about the rotation axis, the base material may be pushed in an axial direction of the needle grooves by centrifugal force.

The method may further include, prior to the forming of the subatmospheric pressure in the mold, in advance rotating the mold filled with the base material about the rotation axis.

The rotation axis may be perpendicular to an axial direction of the needle grooves.

Another aspect of the present disclosure provides an apparatus for manufacturing a microneedle patch including: a mold having a plurality of needle grooves into which a base material is injected; a pressure module configured to create a subatmospheric pressure in the mold; a rotation module configured to rotate the mold about a rotation axis; and a drying module configured to dry the base material filled in the needle grooves.

After bubbles in the base material are removed or gas between the base material and the needle grooves is removed by the pressure module, the base material may be pushed in an axial direction of the needle grooves by centrifugal force generated by the rotation module being driven.

Another aspect of the present disclosure provides a method of manufacturing a microneedle patch including: filling a mold having a plurality of needle grooves with a buffer solution; arranging a base material on the needle grooves; diffusing the base material into the buffer solution; and drying the mold.

In the diffusing of the base material into the buffer solution, the base material may be injected into the needle grooves.

The buffer solution may dissolve the base material.

The buffer solution may include water and the base material may include hyaluronic acid.

An effective ingredient may be included in the base material.

In the drying of the mold, the buffer solution may be removed, and thus the base material may be hardened in the needle grooves.

Another aspect of the present disclosure provides a microneedle patch including a base and a plurality of microneedles arranged on the base, wherein the microneedles are formed by causing a base material to be dissolved in a buffer solution and then drying the buffer solution.

The buffer solution may include water and the base material may include hyaluronic acid.

An effective ingredient may be included in the base material.

Other aspects, features, and advantages other than those described above will be apparent from the following drawings, claims, and detailed description.

### MODE OF DISCLOSURE

Hereinafter, features and functions of the present disclosure will be described in detail with reference to embodiments illustrated in the accompanying drawings.

As the present disclosure allows for various changes and numerous embodiments, particular embodiments will be illustrated in the drawings and described in detail. The effects and features of the present disclosure and methods of achieving them will become clear with reference to the embodiments described in detail below with the drawings. However, the present disclosure is not limited to the embodiments disclosed below, and may be implemented in various forms.

Hereinafter, embodiments of the present disclosure will be described in detail with reference to the accompanying drawings, and the same or corresponding components will be denoted by the same reference numerals when described with reference to the accompanying drawings, and thus their descriptions that are already provided will be omitted.

While such terms as "first," "second," etc., are used only to distinguish one component from another, and such components must not be limited by these terms.

The singular expression also includes the plural meaning as long as it is not inconsistent with the context.

The terms "comprises," "includes," or "has" used herein specify the presence of stated features or elements, but do not preclude the presence or addition of one or more other features or elements.

For ease of description, the magnitude of components in the drawings may be exaggerated or reduced. For example, the magnitude and thickness of each component in the drawings is illustrated for ease of description, and the present disclosure is not limited to the drawings.

When a certain embodiment may be differently implemented, a specific process sequence may be performed differently from the sequence described herein. For example, two processes, which are successively described herein, may be substantially simultaneously performed, or may be performed in a process sequence opposite to a described process sequence.

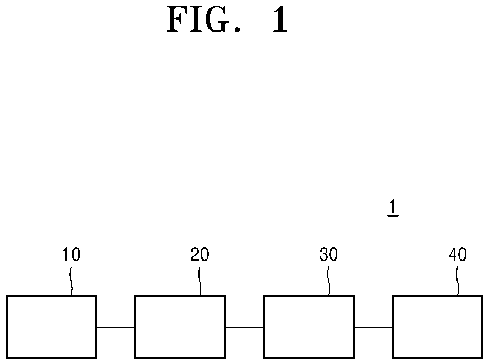

FIG. 1 is a diagram illustrating an apparatus 1 for manufacturing a microneedle patch, according to an embodiment of the present disclosure.

Referring to FIG. 1, the apparatus 1 for manufacturing a microneedle patch may include an injection module 10, a pressure module 20, a rotation module 30, and a drying module 40.

Although FIG. 1 illustrates that the injection module 10, the pressure module 20, the rotation module 30, and the drying module 40 are sequentially arranged in the apparatus 1 for manufacturing a microneedle patch, the present disclosure is not limited thereto, and the arrangement of the components of the apparatus 1 for manufacturing a microneedle patch may be variously modified according to a process of manufacturing a microneedle patch.

For example, in the apparatus 1 for manufacturing a microneedle patch, at least one of the injection module 10, the pressure module 20, the rotation module 30, and the drying module 40 may be provided in plural. As another example, in the apparatus 1 for manufacturing a microneedle patch, the rotation module 30 may be arranged prior to the pressure module 20.

In a microneedle patch 100 manufactured by the apparatus 1 for manufacturing a microneedle patch, a plurality of microneedles 120 may be arranged on a base 110. The microneedle patch 100 may be attached to the skin of a patient to deliver a drug or a cosmetic material.

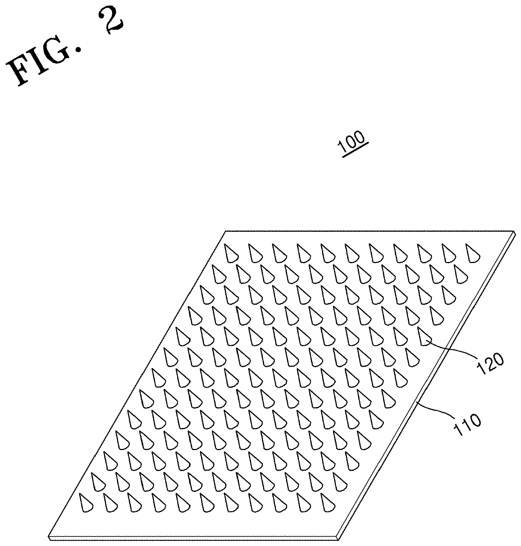

FIG. 2 is a perspective view of the microneedle patch 100 manufactured by the apparatus 1 for manufacturing a microneedle patch of FIG. 1.

Referring to FIG. 2, the microneedle patch 100 may include the base 110 and the microneedles 120.

The plurality of microneedles 120 may be provided on one surface of the base 110, and may be supported by the the base 110. The one surface of the base 110 may come into contact with skin, and the other surface of the base 110 may be exposed to the outside.

The base 110 may be removed after the microneedles 120 are inserted into the skin. For example, the base 110 may be removed from the skin by a user applying a force. As another example, a portion at which the base 110 and the microneedle 120 are coupled to each other first dissolves, and thus the base 110 may be removed after a certain period of time has elapsed after the microneedle patch 100 is attached to the skin. As yet another example, the base 110 may dissolve after a long period of time has elapsed after the microneedle patch 100 is attached to the skin. As yet another example, the base 110 may be removed by the user coating a material for dissolution.

In an embodiment, the base 110 may include any one of materials included in the microneedle 120. The base 110 may include a biodegradable material similarly to the microneedle 120.

In an alternative embodiment, the base 110 may include a physiologically active substance. After attaching the microneedle patch 100 to the skin, an effective drug may be effectively delivered to a patient by the physiologically active substance released from the base 110. In addition, the base 110 and the microneedles 120 may be easily separated from each other by the physiologically active substance released from the base 110.

In an embodiment, the base 110 may have a property of dissolving later than does the closest layer of the microneedle 120, i.e., a layer that is farthest away from the tip of the microneedle 120. Because a portion of the microneedle 120, which is adjacent to the base 110, dissolves the fastest, the base 110 may be easily separated from the microneedle 120.

In an embodiment, the base 110 may include a water-soluble polymer. The base 110 may be formed of a water-soluble polymer or may include other additives (e.g., disaccharides, etc.). In addition, it is preferable that the base 110 does not include a drug or an effective ingredient.

The base 110 may include a biocompatible material. A biocompatible material selected as a base material of the microneedle 120, which will be described below, may also be selected as a base material of the base 110.

The plurality of microneedles 120 may be formed to protrude from the surface of the base 110. The microneedle 120 may be formed of a base material BM, which may include a biocompatible material and an additive.

The biocompatible material may include at least any one of carboxymethyl cellulose (CMC), hyaluronic acid (HA), alginic acid, pectin, carrageenan, chondroitin sulfate, dextran sulfate, chitosan, polylysine, carboxymethyl chitin, fibrin, agarose, pullulan, polyanhydride, polyorthoester, polyetherester, polyesteramide, polybutyric acid, polyvaleric acid, polyacrylate, an ethylene-vinyl acetate polymer, acryl-substituted cellulose acetate, polyvinyl chloride, polyvinyl fluoride, polyvinyl imidazole, chlorosulphonate polyolefins, polyethylene oxide, polyvinylpyrrolidone (PVP), hydroxypropyl methylcellulose (HPMC), ethylcellulose (EC), hydroxypropyl cellulose (HPC), cyclodextrin, maltose, lactose, trehalose, cellobiose, isomaltose, turanose, and lactulose, or at least any one of a copolymer of monomers forming such polymers and cellulose.

The additive may include at least any one of trehalose, oligosaccharide, sucrose, maltose, lactose, cellobiose, HA, alginic acid, pectin, carrageenan, chondroitin sulfate, dextran sulfate, chitosan, polylysine, collagen, gelatin, carboxymethyl chitin, fibrin, agarose, PVP, polyethylene glycol (PEG), polymethacrylate, HPMC, EC, HPC, carboxymethyl cellulose, cyclodextrin, gentiobiose, cetrimide (alkyltrimethylammonium bromide), cetrimonium bromide (hexadecyltrimethylammonium bromide (CTAB)), gentian violet, benzethonium chloride, docusate sodium salt, a SPAN-type surfactant, polysorbate (Tween), sodium lauryl sulfate (sodium dodecyl sulfate (SDS)), benzalkonium chloride, and glyceryl oleate.

The term "hyaluronic acid (HA)" is used herein to encompass hyaluronic acid, hyaluronates (e.g., sodium hyaluronate, potassium hyaluronate, magnesium hyaluronate, and calcium hyaluronate), and mixtures thereof. The term "hyaluronic acid (HA)" is also used here to encompass cross-linked hyaluronic acid and/or non-cross-linked hyaluronic acid.

According to an embodiment of the present disclosure, the molecular weight of the HA is 2 kDa to 5000 kDa.

According to another embodiment of the present disclosure, the molecular weight of the HA is 100 kDa to 4500 kDa, 150 kDa to 3500 kDa, 200 kDa to 2500 kDa, 220 kDa to 1500 kDa, 240 kDa to 1000 kDa, or 240 kDa to 490 kDa.

The CMC used herein may be known CMC with various molecular weights. For example, the average molecular weight of the CMC used herein is 90,000 kDa, 250,000 kDa, or 700,000 kDa.

The disaccharides may be sucrose, lactulose, lactose, maltose, trehalose, cellobiose, or the like, and may particularly include sucrose, maltose, and trehalose.

In an alternative embodiment, the microneedle 120 may include an adhesive. The adhesive is at least one adhesive selected from the group consisting of silicone, polyurethane, HA, a physical adhesive (Gecko), polyacryl, EC, hydroxymethyl cellulose, ethylene-vinyl acetate, and polyisobutylene.

In an alternative embodiment, the microneedle 120 may further include a metal, a polymer, or an adhesive.

The microneedle 120 may include an effective ingredient EM. The microneedle 120 may include the effective ingredient EM in at least a portion thereof, and the effective ingredient EM may be a pharmaceutically, medically, or cosmetically effective ingredient. For example, the effective ingredient includes, but is not limited to, a protein/peptide medicine, and may include at least one of a hormone, a hormone analogue, an enzyme, an enzyme inhibitor, a signal transduction protein or a portion thereof, an antibody or a portion thereof, a single-chain antibody, a binding protein or a binding domain thereof, an antigen, an adherent protein, a structural protein, a regulatory protein, a toxic protein, a cytokine, a transcription regulator, a blood coagulation factor, and a vaccine. In detail, the protein/peptide medicine may include at least one of insulin, insulin-like growth factor 1 (IGF-1), growth hormone, erythropoietin, granulocyte colony-stimulating factors (G-CSFs), granulocyte/macrophage colony-stimulating factors (GM-CSFs), interferon alpha, interferon beta, interferon gamma, interleukin-1 alpha and beta, interleukin-3, interleukin-4, interleukin-6, interleukin-2, epidermal growth factors (EGFs), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosine, triptorelin, bivalirudin, carbetocin, cyclosporine, exedine, lanreotide, luteinizing hormone-releasing hormone (LHRH), nafarelin, parathyroid hormone, pramlintide, enfuvirtide (T-20), thymalfasin, and ziconotide. In addition, the effective ingredient EM may be a cosmetic ingredient such as a skin lightening agent, a filler, a wrinkle reducing agent, or an antioxidant.

In an embodiment, the effective ingredient EM may be colloid particles dispersed in a solvent forming the microneedle 120. The particles themselves may be the effective ingredient EM or may include a coating material carrying the effective ingredient EM.

The effective ingredient EM may be intensively distributed in a partial layer of the microneedle 120. That is, the effective ingredient EM may be at a certain height in the microneedle 120, and thus, the effective ingredient EM may be effectively delivered.

In another embodiment, the effective ingredient EM may be dissolved in the microneedle 120. The effective ingredient EM may be dissolved in the base material of the microneedle 120, such as the biodegradable materials described above, to constitute the microneedle 120. The effective ingredient EM may be uniformly dissolved in the base material and may be intensively distributed at a certain height of the microneedle 120, like the above-described particles.

In an embodiment, the microneedle patch 100 may have a plurality of effective ingredients EM in respective regions thereof. A first group of microneedles among the plurality of microneedles may include a first effective ingredient among the plurality of effective ingredients, and a second group of microneedles different from the first group may include a second effective ingredient among the plurality of effective ingredients.

In an embodiment, the pharmaceutically, medically, or cosmetically effective ingredient EM may be coated on the microneedle 120. The effective ingredients EM may be coated on the entire microneedle 120 or only a portion of the microneedle 120. Alternatively, in the microneedle 120, the first effective ingredient may be coated on a portion of a coating layer, and the second effective ingredient may be coated on another portion of the coating layer.

The microneedle 120 may have various shapes. The microneedle 120 may have a conical shape. For example, the microneedle 120 may have a polygonal shape such as a triangular pyramid shape, or a quadrangular pyramid shape.

The microneedle 120 may have a layered structure. The microneedle 120 may have a plurality of stacked layers. The number of layers constituting the microneedle 120 is not limited to a certain number.

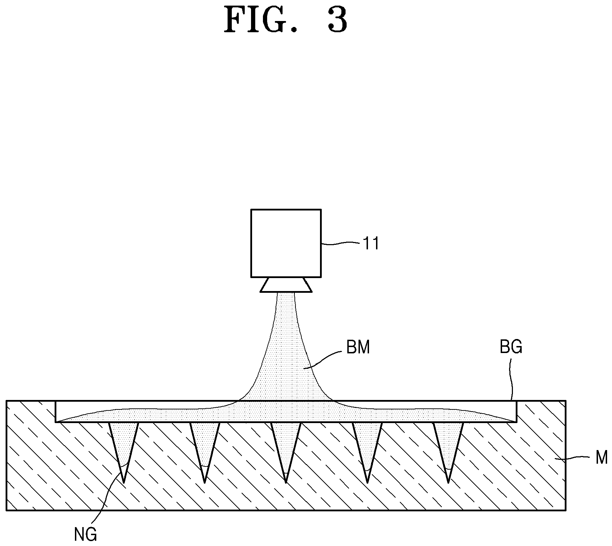

FIG. 3 is a diagram illustrating the base material BM being injected from the injection module 10 of FIG. 1.

Referring to FIG. 3, the base material BM may be injected into a mold M from the injection module 10.

The mold M includes a base groove BG and a plurality of needle grooves NG. The base groove BG is a region in which the base 110 is formed, the needle groove NG is a region in which the microneedle 120 is formed, and the base material BM is injected into the needle groove NG.

The base material BM is arranged on the needle groove NG and is injected into the needle groove NG. The base material BM may be injected into the mold M in various ways.

For example, the base material BM may be liquid, and may be sprayed from a nozzle 11 to be injected into the mold M.

Alternatively, the base material BM may be injected into the needle groove NG by depositing droplets of the base material BM into the needle groove NG.

As another example, the base material BM may be in a gel form, and may be placed on the mold M by using a spatula. Thereafter, the base material BM may be pressed by a squeezing device (not shown) to be injected into the needle groove NG.

The base material BM injected into the mold M may have bubbles formed therein. The bubbles may be formed when preparing the base material BM and when injecting the base material BM into the mold M. In addition, gas may be stored between the base material BM and the needle groove NG. When injecting the base material BM into the mold, the gas may not be completely removed and remain in spaces between the needle grooves NG and the base material BM.

However, when the pressure module 20 or the rotation module 30, which will be described below, is driven, the bubbles in the base material BM may be removed, and the needle grooves NG may be completely filled with the base material BM.

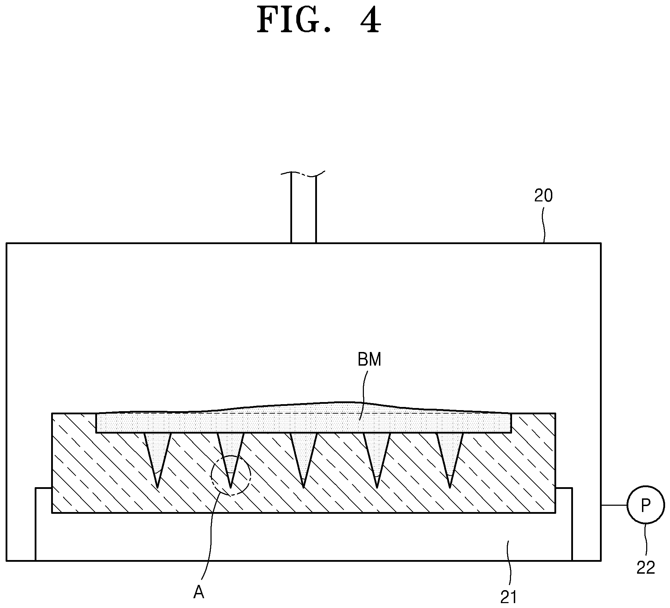

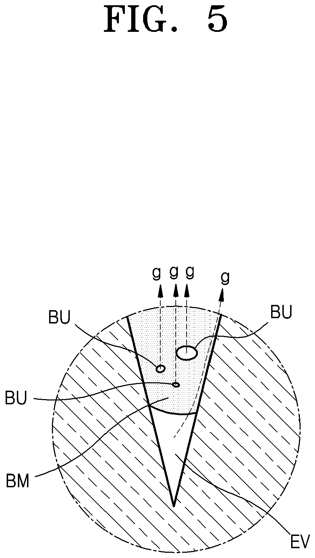

FIG. 4 is a diagram illustrating driving of the pressure module 20 of FIG. 1, and FIG. 5 is an enlarged view of region A of FIG. 4.

Referring to FIGS. 4 and 5, the pressure module 20 may be driven to remove gas remaining in the needle groove NG or to remove bubbles within the base material BM.

Hereinafter, driving the pressure module 20 to cause the mold M to be at a low pressure means causing the mold M to be at a relatively low pressure in order to remove the gas in the needle groove NG or remove the bubbles within the base material BM. That is, creating an atmosphere with a subatmospheric pressure in the mold M means creating a low-pressure atmosphere in order to remove the gas included in the base material BM or to remove the gas remaining between the mold M and the base material BM.

For example, driving the pressure module 20 to cause the mold M to be at a low pressure may mean causing a pressure chamber to be at a subatmospheric pressure. In addition, driving the pressure module 20 to cause the mold M to be at a low pressure may mean setting the internal pressure of the pressure chamber to 1 atm or less. In addition, driving the pressure module 20 to cause the mold M to be at a low pressure may include setting the internal pressure of the pressure chamber to 1 mbar or less so as to create a substantially vacuum environment.

The mold M is placed on a support 21 of the pressure module 20, and then a pressure pump 22 is driven to cause the inside of the pressure chamber to be at a low pressure. Here, the low pressure may be set to a subatmospheric pressure. Because the needle groove NG of the mold M is also at a relatively low pressure, gas g in bubbles BU in the base material BM is removed. In addition, the gas g in the spaces between the base material BM and the needle grooves NG is also removed.

That is, the pressure module 20 may remove the gas g in the base material BM or remove the gas g between the base material BM and the needle grooves NG, thereby increasing the quality of the microneedles 120.

In an embodiment, after the gas g is removed, the driving of the pressure pump 22 is stopped to release the state in which the mold M is at the subatmospheric pressure. As the low-pressure state of the mold M is released, the bubbles BU in the base material BM are maintained as empty spaces, and a space between the surface of the needle groove NG and the base material BM is also maintained as an empty space.

In another embodiment, the driving of the pressure pump 22 may be maintained even after the gas g is removed, such that the mold M is maintained at the subatmospheric pressure. Thereafter, the rotation module 30 may be driven while maintaining the low-pressure state so as to fill the empty spaces with the base material BM.

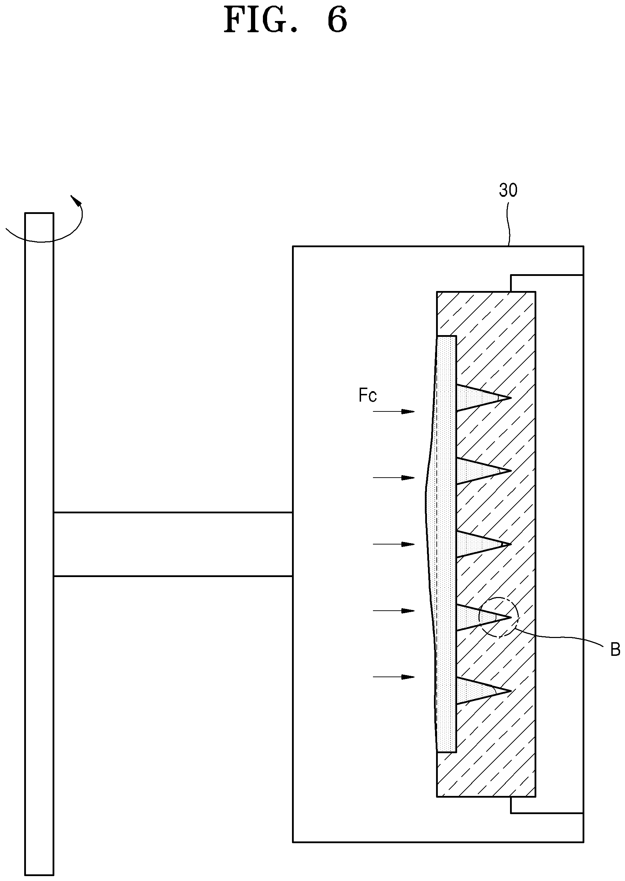

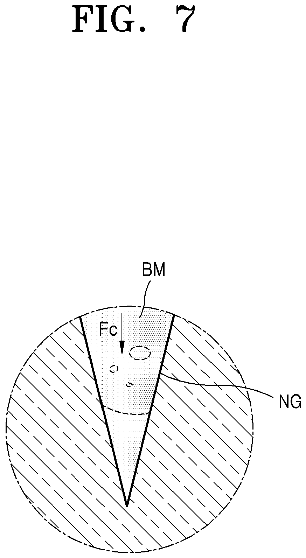

FIG. 6 is a diagram illustrating driving of the rotation module 30, and FIG. 7 is a diagram illustrating filling of the mold M with the base material BM in region B of FIG. 6.

Referring to FIGS. 6 and 7, the rotation module 30 rotates the mold M about a rotation axis RX to provide centrifugal force Fc to the mold M.

The rotation axis RX is different from the longitudinal direction of the needle groove NG. In order to provide the centrifugal force Fc in the axial direction of the needle groove NG, the rotation axis RX and the longitudinal direction of the needle groove NG are set to be different from each other. Preferably, the rotation axis RX and the longitudinal direction of the needle groove NG may be set to be perpendicular to each other.

The centrifugal force Fc is generated in the longitudinal direction of the needle groove NG, and thus, the base material BM is completely filled in the needle groove NG.

Due to the centrifugal force Fc, the base material BM is pushed to the end of the needle groove NG. At this time, the base material BM is filled in regions of the needle grooves NG, which are not yet filled with the base material BM. In addition, the base material BM becomes dense by the centrifugal force Fc, and thus, the bubbles BU therein are removed.

Even when the low-pressure state of the mold M is released, the gas g is removed from regions in which the base material BM is not filled and the bubbles BU in the mold M, and thus, the pressure in the mold is still lower than that of the outside. At this time, when the centrifugal force Fc is applied to the base material BM, the base material BM may be rapidly and easily filled in the needle grooves NG and the bubbles BU.

The rotation module 30 may provide the centrifugal force Fc to the mold M, and thus, the needle grooves NG may be completely filled with the base material BM. Accordingly, by using the mold M, the fine microneedles 120 may be precisely manufactured, and pores may be removed from the inside of the microneedles 120.

In another embodiment, the rotation module 30 may be driven prior to driving of the pressure module 20. That is, the base material BM is injected into the mold M in the injection module 10, and then the rotation module 30 is driven. The needle groove NG may be filled with the base material BM up to the end thereof by the centrifugal force Fc generated by the rotation module 30.

Thereafter, the above-described pressure chamber is driven to remove the gas g in the bubbles BU in the base material BM or the gas g between the base material BM and the needle grooves NG. Thereafter, the rotation module 30 may be driven again, and thus, the needle grooves NG may be completely filled with the base material BM.

Referring back to FIG. 1, the drying module 40 dries the base material BM. As the base material BM is completely dried, the microneedles 120 are finished. After the base material BM is completely dried, the microneedle patch 100 is separated from the mold M.

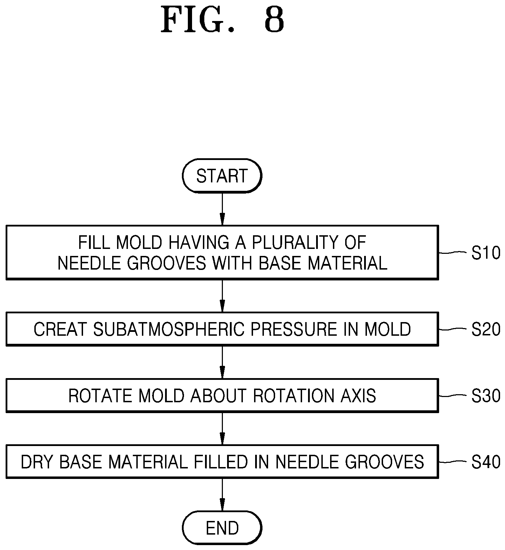

FIG. 8 is a flowchart of a method of manufacturing a microneedle patch, according to another embodiment of the present disclosure.

Referring to FIG. 8, the method of manufacturing a microneedle patch includes filling a mold having a plurality of needle grooves with a base material (S10), creating a subatmospheric pressure in the mold (S20), rotating the mold about a rotation axis (S30), and drying the base material filled in the needle grooves (S40).

In the filling of the mold having the plurality of needle grooves with the base material (S10), the base material BM is injected into the mold M.

In an alternative embodiment, when injecting the base material BM into the mold M, a chamber in which the mold M is installed may be set to be at a low pressure. That is, also in the filling of the mold having the plurality of needle grooves with the base material (S10), the inside of the chamber may be maintained at a subatmospheric pressure. Accordingly, the formation of bubbles in the base material BM may be minimized.

In a process of preparing the base material BM, gas such as air may be stored in the bubbles BU in the base material BM. Even when the base material BM is injected into the needle grooves NG, the bubbles BU may still exist in the needle grooves NG.

The gas g remaining in the bubbles BU in the base material BM may seriously degrade the quality of the microneedles 120. Because the microneedle 120 is to be implanted into the skin of a patient, the microneedle 120 should not contain foreign substances including gas. When the gas g in the bubbles BU is injected into the patient, the safety of the patient may be threatened.

Because the needle groove NG has a significantly low volume while the base material BM has a certain viscosity, it is difficult to completely fill the needle groove NG with the base material BM. Even when the base material BM is placed in the needle groove NG, the gas g may remain between the base material BM and the surface of the needle groove NG.

An empty space between the needle groove NG and the base material BM seriously degrades the quality of the microneedle patch 100. Because the microneedle 120 is required to be inserted through the skin of the patient, the microneedle 120 needs to have a sharpened tip. However, the empty space between the needle groove NG and the base material BM causes the end of the microneedle 120 to be blunt, thus, it is difficult for the microneedle 120 to be attached onto the skin of the patient, and accordingly, the drug delivery effect is reduced.

In order to prevent degradation in quality in the manufacture of the microneedle patch 100, the following operations are performed.

In the creating of the subatmospheric pressure in the mold (S20), the bubbles BU in the base material BM filled in the needle groove NG may be removed, or the gas g between the base material BM and the needle groove NG may be removed. In operation S20, the subatmospheric pressure may be created in the mold M, i.e., the internal pressure of the chamber in which the mold M is installed may be set to a pressure lower than 1 atm, so as to remove the gas g in the bubbles BU in the base material BM and remove the gas g remaining in the needle groove NG.

When the mold M is at the subatmospheric pressure, the gas g in the bubbles BU is discharged to the outside of the mold M. In addition, when the mold M is at the subatmospheric pressure, the gas g stored between the base material BM and the surface of the needle groove NG is also discharged to the outside of the mold M.

In the rotating of the mold about the rotation axis (S30), the base material BM may be completely filled in the needle grooves NG by generating the centrifugal force Fc in the mold M. In operation S30, the base material BM is pushed in the axial direction of the needle groove NG by the centrifugal force Fc.

In an embodiment, before applying the centrifugal force Fc to the mold M, the low-pressure state of the mold M is released. Thereafter, when the mold M is rotated about the rotation axis RX, the base material BM is deeply injected into the needle grooves NG by the centrifugal force Fc.

In another embodiment, the mold M may be rotated about the rotation axis RX while the mold M is maintained at a subatmospheric pressure. Because the mold M is continuously maintained in the low-pressure atmosphere, the base material BM is deeply injected into the needle grooves NG by the centrifugal force Fc.

Because the rotation axis RX and the longitudinal axis of the needle groove NG are different from each other, when the centrifugal force Fc is applied to the base material BM, the needle groove NG is completely filled with the base material BM up to the end thereof. When the centrifugal force Fc generated by the rotation module 30 is provided to the mold M, the space from which the gas g is removed in the pressure chamber is filled with the base material BM.

In the drying of the base material filled in the needle grooves (S40), the base material BM filled in the mold M is dried to form the microneedles 120. Thereafter, the manufactured microneedle 120 may be removed from the mold M.

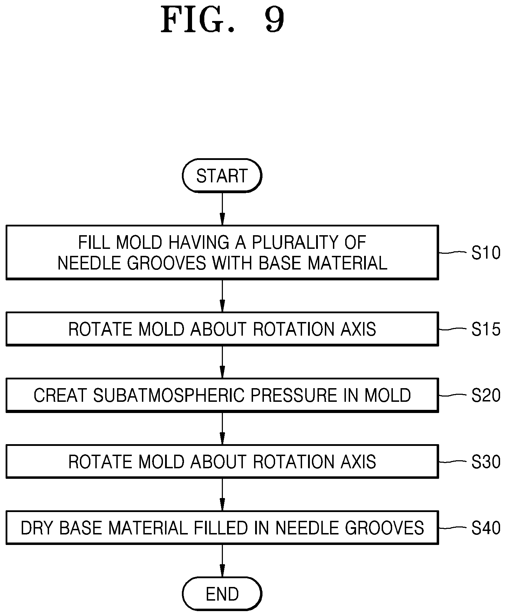

FIG. 9 is a flowchart of a method of manufacturing a microneedle patch, according to another embodiment of the present disclosure.

Referring to FIG. 9, the method may further include rotating the mold filled with the base material (S15), before the creating of the subatmospheric pressure in the mold.

That is, after the base material BM is injected into the mold M, the mold M is rotated about the rotation axis RX. Consequently, the base material BM is pushed deep into the needle grooves NG by centrifugal force.

When the centrifugal force is primarily applied to the mold M filled with the base material BM, the base material BM may be uniformly distributed into the needle grooves NG by the centrifugal force. When the inside of the chamber in which the mold M is installed is set to be at a low pressure, the gas g is removed from the needle grooves NG or the base material BM. When the centrifugal force is secondarily applied to the mold M, the base material BM is completely brought into close contact with the needle grooves NG by the centrifugal force, and thus the quality of the microneedles 120 may be improved.

In the method of manufacturing a microneedle patch according to another embodiment of the present disclosure, the filling of the mold having the plurality of needle grooves with the base material (S10), the creating of the subatmospheric pressure in the mold (S20), and the rotating of the mold about the rotation axis (S30) may be performed in the same chamber.

Here, during both of the filling of the mold having the plurality of needle grooves with the base material (S10) and the rotating of the mold about the rotation axis (S30), the subatmospheric pressure may be created and maintained in the mold.

Because the low-pressure atmosphere is maintained even when filling the mold M with the base material BM, the formation of bubbles in the injection process may be minimized. In addition, because the low-pressure atmosphere is maintained even when rotating the mold M about the rotation axis RX, the base material BM may be densely injected into the end of the needle groove NG.

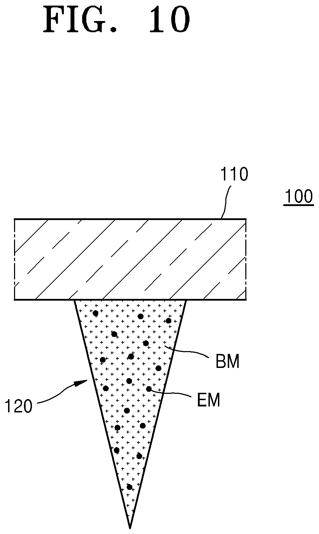

FIGS. 10 to12 are diagrams illustrating microneedle patches manufactured by using the method of manufacturing a microneedle patch of the present disclosure.

Referring to FIG. 10, the microneedle patch 100, which is manufactured by using the apparatus 1 for manufacturing a microneedle patch or the method of manufacturing a microneedle patch, may include the base 110 and the microneedle 120 having a single-layer structure. The microneedle 120 may include an effective ingredient EM therein.

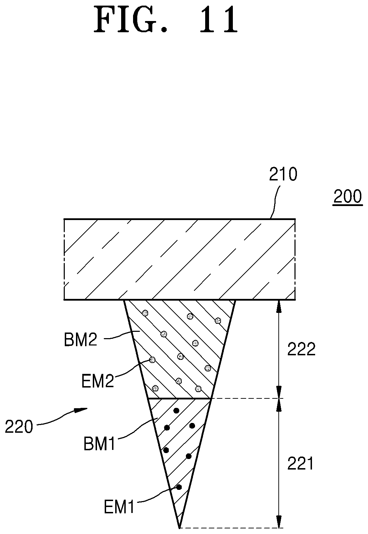

Referring to FIG. 11, a microneedle patch 200 may be manufactured by using the apparatus 1 for manufacturing a microneedle patch or the method of manufacturing a microneedle patch described above. The microneedle patch 200 may include a base 210 and a multi-layered microneedle 220.

The multi-layered microneedle patch 200 may be manufactured by driving the apparatus 1 for manufacturing a microneedle patch a plurality of times or by performing the method of manufacturing a microneedle patch a plurality of times.

In detail, a first layer 221 is formed by injecting a first base material into the needle grooves NG, primarily creating a subatmospheric pressure in the mold M, rotating the mold M, and drying the first base material.

Thereafter, a second layer 222 is formed by injecting a second base material onto the first layer 221, secondarily creating a subatmospheric pressure in the mold M, rotating the mold M, and drying the second base material.

Here, the subatmospheric pressure primarily formed in the mold M and the subatmospheric secondarily formed in the mold M may be set to be different from each other. For example, the secondarily created subatmospheric pressure may be less than the primarily created subatmospheric pressure, and thus, the gas g remaining in the base material BM or the needle grooves NG may be completely removed.

In the microneedle patch 200, the microneedle 220 arranged on one surface of the base 210 has a multi-layer structure, and thus may accurately deliver the effective ingredient EM to a target point. Because the microneedle 220 includes a plurality of layers, the effective ingredient EM may be included in each layer. For example, a first effective ingredient EM1 may be included in the first layer 221, and a second effective ingredient EM2 may be included in the second layer 222. Consequently, in the microneedle patch 200, the depth at which each effective ingredient is to be activated may be adjusted according to the height of each layer. That is, the microneedle patch 200 may deliver the first and second effective ingredients EM 1 and EM2 to any one of an epidermis, a dermis, subcutaneous fat, and muscle, respectively.

Because the microneedle patch 200 has the multi-layer structure, the biodegradation rates of the layers may be set to be different from each other. In the microneedle 220, the decomposition rates of the first layer 221 and the second layer 222 may be set to be different from each other, and thus, the first effective ingredient EM1 and the second effective ingredient EM2 may be activated at different points of time.

Because the microneedle patch 200 has the multi-layer structure, and the strengths of the layers may be set to be different from each other. By setting the strength of the first layer 221 to be greater than the strength of the second layer 222, the microneedle 220 may be easily inserted into the skin.

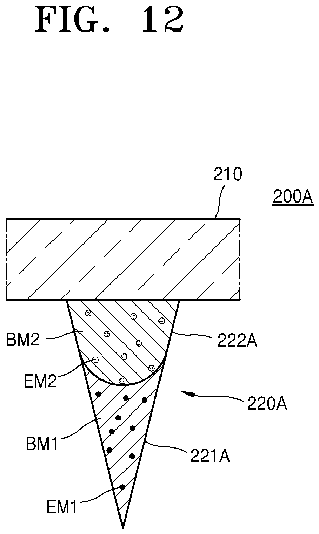

Referring to FIG. 12, a microneedle patch 200' may be manufactured by using the apparatus 1 for manufacturing a microneedle patch or the method of manufacturing a microneedle patch described above. The microneedle patch 200' may include the base 210 and a multi-layered microneedle 220'.

The microneedle 220' may include a first layer 221' and a second layer 222'. The first layer 221' is formed by injecting a first base material into the mold M. In a drying process, as the first base material is dried, a curved surface may be formed in the first layer 221'. Thereafter, a second base material is injected onto the first layer 221' to form the second layer 222'.

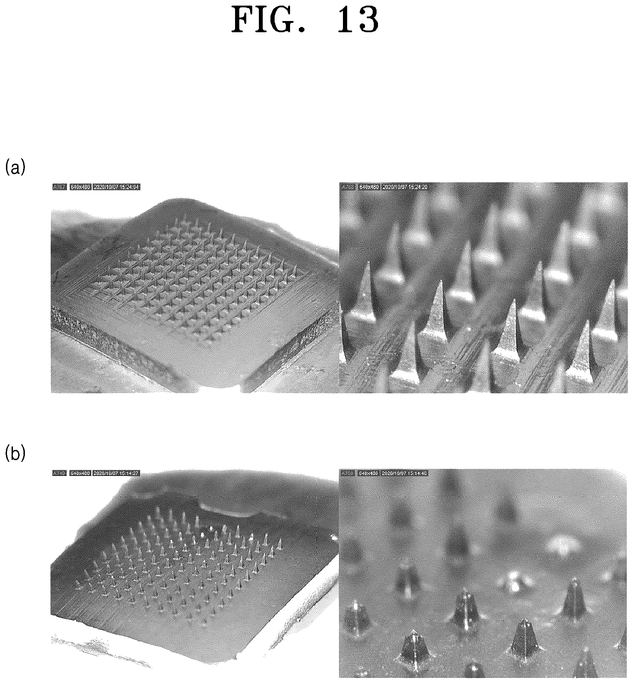

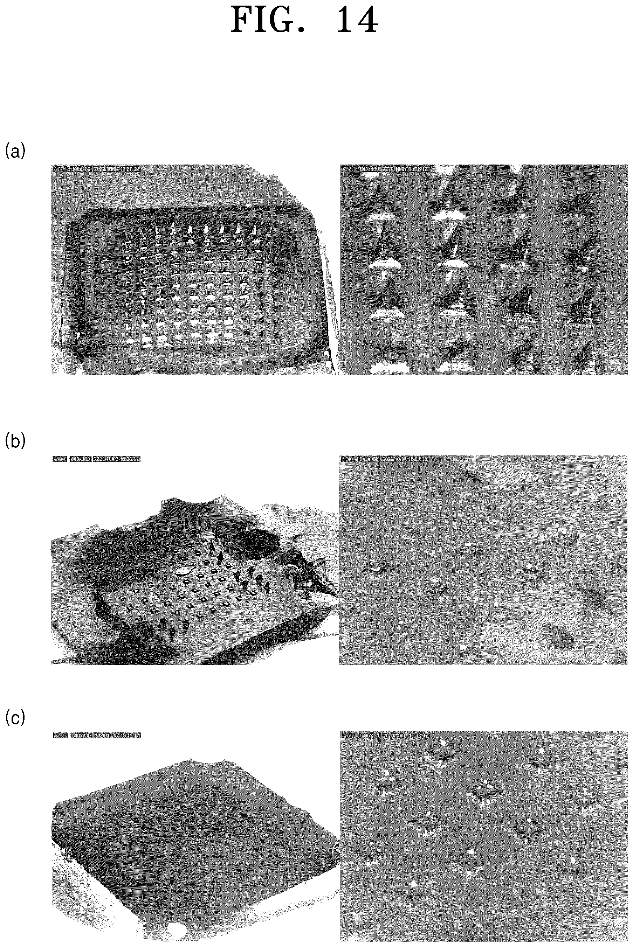

FIGS. 13 and 14 are diagrams showing microneedle patches manufactured by the method of manufacturing a microneedle patch of the present disclosure and comparative examples.

In FIG. 13, (a) shows a microneedle patch, which was manufactured by using the method of manufacturing a microneedle patch according to an embodiment of the present disclosure, and (b) shows a microneedle patch, which is a comparative example and was manufactured with the operation of rotating a mold to generate centrifugal force but without the operation of creating a low pressure, wherein the microneedle patches were manufactured by using 200 kDa 10 % HA as a base material.

For manufacturing the microneedle patch of (a), 200 kDa 10 % HA was injected into the mold, and then the mold was caused to be at a low pressure (less than 1 mbar) for 10 minutes. The mold was then rotated at 500 rpm for 30 seconds to apply centrifugal force to the mold. Thereafter, the mold was dried in an oven for 48 hours.

For manufacturing the microneedle patch of (b), 200 kDa 10% HA was injected into the mold, and then the mold was rotated at 500 rpm for 30 seconds to apply centrifugal force to the mold. Thereafter, the mold was dried in an oven for 48 hours. In the comparative example, the mold was not caused to be at a low pressure.

In the microneedle patch according to the method of manufacturing a microneedle patch of the present disclosure, the tip of the microneedle is precisely formed to be significantly sharp. On the other hand, in the microneedle patch of the comparative example, the tip of the microneedle is formed to be blunt, it is difficult for the microneedle patch to be attached to the skin of the patient. In the comparative example, the mold was not caused to be at a subatmospheric pressure, thus the HA was not completely injected into the needle grooves of the mold, gas remained in the injected HA, and thus, the quality of the microneedle patch is low.

In FIG. 14, (a) shows a microneedle patch, which was manufactured by using the method of manufacturing a microneedle patch according to an embodiment of the present disclosure, (b) shows a microneedle patch, which is a first comparative example and was manufactured with the operation of creating a low pressure but without the operation of rotating a mold to generate centrifugal force, and (c) shows a microneedle patch, which is a second comparative example and was manufactured with the operation of rotating the mold to generate centrifugal force but without the operation of creating a low pressure, wherein the microneedle patches were manufactured by using 1.4 MDa 10 % HA as a base material.

The 1.4 MDa 10% HA of FIG. 14 has a higher viscosity and a higher strength than those of the 200 kDa 10% HA of FIG. 13. Accordingly, it is relatively more difficult to manufacture microneedles by using 1.4 MDa 10 % HA, which has a high viscosity, than by using 200 kDa 10 % HA.

For manufacturing the microneedle patch of (a), 1.4 MDa 10 % HA was injected into the mold, and then the mold was caused to be at a low pressure (less than 1 mbar) for 10 minutes. The mold was then rotated at 2,000 rpm for 2 minutes to apply centrifugal force to the mold. Thereafter, the mold was dried in an oven for 48 hours.

For manufacturing the microneedle patch of (b), 1.4 MDa 10 % HA was injected into the mold, and then the mold was caused to be at a low pressure (less than 1 mbar) for 10 minutes. Thereafter, the mold was dried in an oven for 48 hours.

For manufacturing the microneedle patch of (c), 1.4 MDa 10% HA was injected into the mold, and then the mold was rotated at 500 rpm for 30 seconds to apply centrifugal force to the mold. The mold was then rotated at 2,000 rpm for 2 minutes to apply centrifugal force to the mold. Thereafter, the mold was dried in an oven for 48 hours.

In the microneedle patch according to the method of manufacturing a microneedle patch of the present disclosure, the tip of the microneedle is precisely formed to be significantly sharp. On the other hand, in the microneedle patches of the first and second comparative examples, the tips of the microneedles are formed to be blunt, it is difficult for the microneedle patches to be attached to the skin of the patient.

The method of manufacturing a microneedle patch according to the present disclosure may increase the quality of the microneedle patch by completely injecting high-molecular-weight HA into needle grooves. Although it is difficult to completely inject 1.4 MDa 10% HA, which has a high molecular weight, into the needle grooves due to the high viscosity of the HA, according to the present disclosure, a subatmospheric pressure and centrifugal force are applied to the mold such that 1.4 MDa 10 % HA is completely injected into the mold, and thus, the quality of the microneedle patch may be improved.

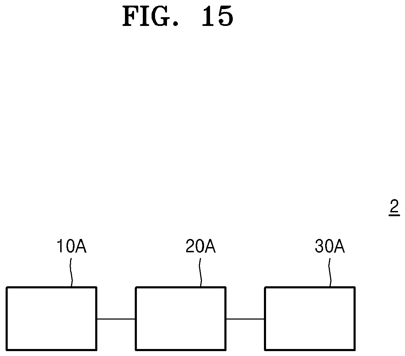

FIG. 15 is a diagram illustrating an apparatus 2 for manufacturing a microneedle patch, according to another embodiment of the present disclosure.

Referring to FIG. 15, an apparatus 2 for manufacturing a microneedle patch may include a first injection module 10A, a second injection module 20A, and a drying module 30A.

Although FIG. 15 illustrates that the first injection module 10A, the second injection module 20A, and the drying module 30A are sequentially arranged in the apparatus 2 for manufacturing a microneedle patch, the present disclosure is not limited thereto, and the arrangement of the components of the apparatus 2 for manufacturing a microneedle patch may be variously modified according to a process of manufacturing a microneedle patch.

For example, in the apparatus 2 for manufacturing a microneedle patch, at least one of the first injection module 10A, the second injection module 20A, and the drying module 30A may be provided in plural. As another example, in the apparatus 2 for manufacturing a microneedle patch, the drying module 30A may be arranged prior to the second injection module 20A.

In an embodiment, the first injection module 10A may inject a buffer solution BS into the mold M, and the second injection module 20A may arrange the base material BM in the mold M. As illustrated in FIG. 15, the first injection module 10A and the second injection module 20A may be provided separately from each other.

In another embodiment, the first injection module 10A may inject both the buffer solution BS and the base material BM into the mold M. The first injection module 10A may be integrated to inject both the buffer solution BS and the base material BM.

The drying module 30A dries the mold M after the mold M is mounted therein. When the drying module 30A is driven, the buffer solution BS may be removed.

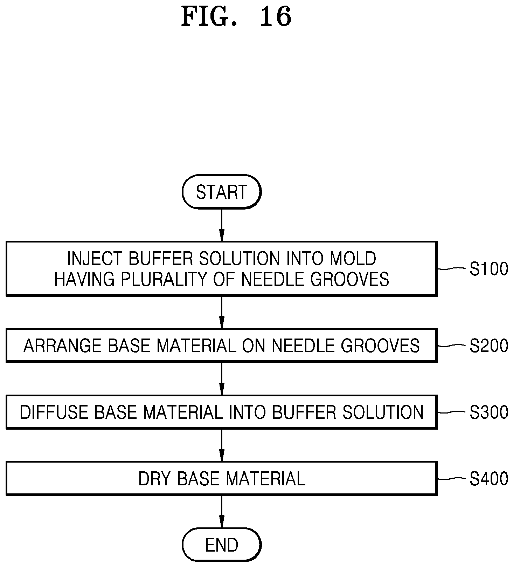

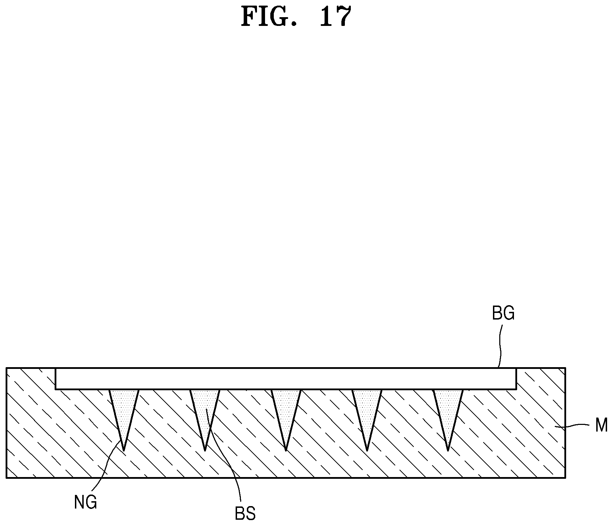

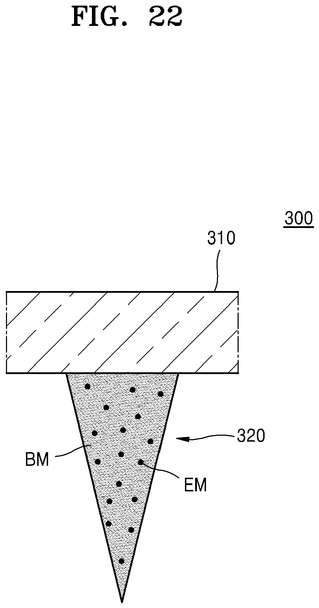

FIG. 16 is a flowchart of a method of manufacturing a microneedle patch according to another embodiment of the present disclosure, FIGS. 17 to 21 are diagrams illustrating operations of the method of manufacturing a microneedle patch of FIG. 16, and FIG. 22 is a diagram illustrating a microneedle patch manufactured by the method of manufacturing a microneedle patch of FIG. 16.

Referring to FIGS. 16 to 22, the method of manufacturing a microneedle patch includes filling a mold having a plurality of needle grooves with a buffer solution (S100), placing a base material above the needle grooves (S200), diffusing the base material into the buffer solution (S300), and drying the mold (S400).

Hereinafter, the buffer solution BS refers to a solution capable of dissolving the base material BM. The buffer solution BS is a material capable of dissolving the base material BM that forms the microneedles. The buffer solution BS has a solubility with respect to the base material BM, and depends on the type of the base material BM.

For example, the buffer solution BS may be a solution 'a' capable of dissolving a base material 'A'. Alternatively, the buffer solution BS may be a solution 'b' capable of dissolving a base material 'B'.

For example, when the base material BM is hyaluronic acid, the buffer solution BS may be water, particularly, purified water.

In an embodiment, the buffer solution BS may not dissolve the effective ingredient EM. The effective ingredient EM is contained in the base material BM, and the buffer solution BS dissolves the base material BM, but does not dissolve the effective ingredient EM. However, the effective ingredient EM may be injected into the needle grooves NG together with the base material BM, and thus arranged inside the microneedles.

In another embodiment, the buffer solution BS may dissolve the effective ingredient EM. The effective ingredient EM is contained in the base material BM, and the buffer solution BS dissolves both the base material BM and the effective ingredient EM. For example, when the buffer solution BS is water and the effective ingredient EM is water-soluble, the effective ingredient EM may be dissolved in the buffer solution BS.

In the filling of the mold having the plurality of needle grooves with the buffer solution (S100), the mold M is filled with the buffer solution BS, and the needle grooves NG are filled with the buffer solution BS (see FIG. 17). The buffer solution BS may be filled in the mold M through the first injection module 10A.

The mold M may have the base groove BG and the needle groove NG. The base 110 may be formed in the base groove BG, and a microneedle 320 may be formed in the needle groove NG.

The buffer solution BS may be fully filled in the needle groove NG.

In an embodiment, the buffer solution BS may be filled in only the plurality of needle grooves NG. In order to form the microneedles 320 first and then form a base 310, the buffer solution BS is injected into only the plurality of needle grooves NG.

In another embodiment, the buffer solution BS may be fully filled in both the needle grooves NG and the base groove BG. In order to manufacture a microneedle patch 300 in which the base 310 and the microneedles 320 are integrated, the buffer solution BS may be fully filled in both the needle grooves NG and the base groove BG, and the base material BM may be filled in the entire mold M.

In another embodiment, the buffer solution BS may be filled in only a portion of the needle groove NG. The microneedle 320 having a multi-layer structure may be sequentially manufactured by injecting the buffer solution BS into the portion of the needle groove NG, and then injecting the buffer solution BS into the remaining portions of the needle groove NG. This will be described in detail below.

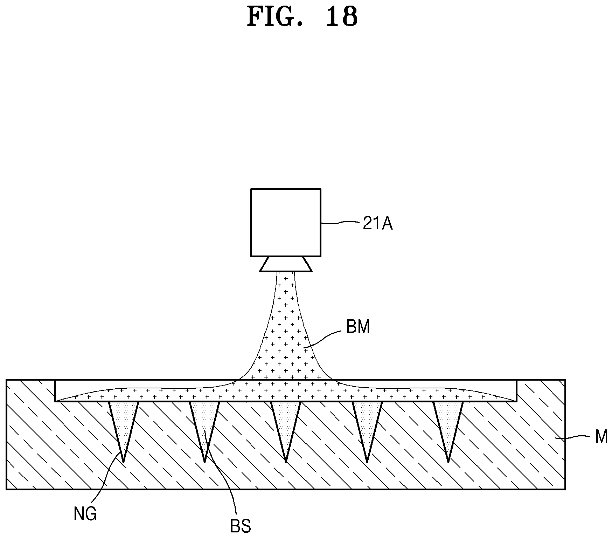

In the placing of the base material on the needle grooves, the base material BM is placed above the mold M (see FIG. 18).

The base material BM is placed on the needle grooves NG such that the base material BM is set to be easily dissolved in the buffer solution BS stored in the needle grooves NG. The base material BM may be injected into the base groove BG by a nozzle 21A of the second injection module 20A.

Because the base material BM has a certain viscosity, it is difficult for the base material BM to be directly injected into the needle grooves NG. However, when the base material BM is dissolved in the buffer solution BS, its fluidity increases, and thus the base material BM may be filled in the needle grooves NG.

In an embodiment, the base material BM is injected into the base groove BG of the mold M. In order for the needle groove NG to be sufficiently filled with the base material BM, the base material BM may be fully filled in the base groove BG or may be filled in the base groove BG in an amount exceeding the capacity of the base groove BG to be convex.

In the diffusing of the base material into the buffer solution (S300), the base material BM may be dissolved in the buffer solution BS.

The base material BM is dissolved in the buffer solution BS and then injected into each of the needle grooves NG.

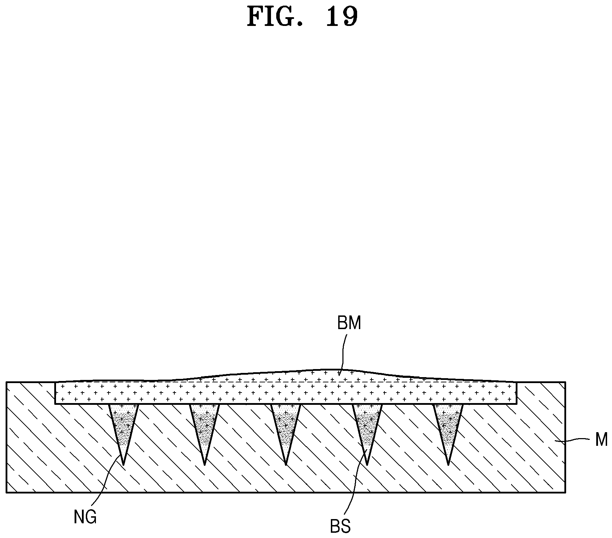

As illustrated in FIG. 19, the base material BM is initially diffused into an upper portion of the needle groove NG, and is gradually diffused into the tip of the needle groove NG.

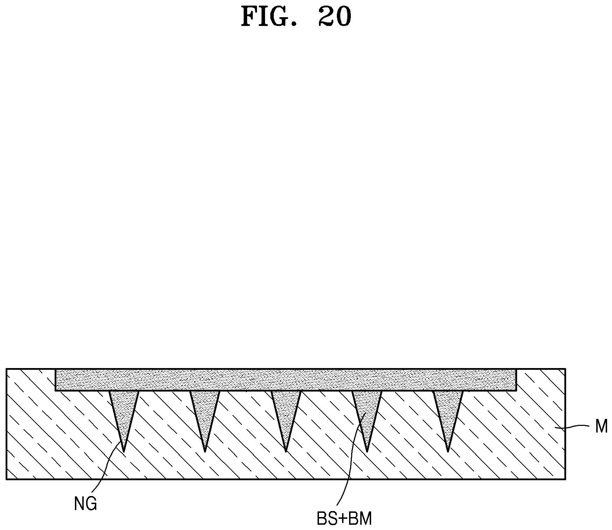

Thereafter, as illustrated in FIG. 20, the base material BM is filled in the entire mold M. When the base material BM is completely dissolved in the buffer solution BS, the concentration and viscosity of the base material BM are decreased.

Because the buffer solution BS is completely filled in the needle groove NG, the base material BM is dissolved in the buffer solution BS and thus is completely filled in the needle groove NG. As the base material BM is completely filled in the needle groove NG, the microneedle 120 may be formed in the shape of the needle groove NG.

In an embodiment, when the base material BM is hyaluronic acid, water, particularly purified water, may be stored in the needle grooves NG as the buffer solution BS. Because hyaluronic acid is dissolved in water, the hyaluronic acid is filled in the needle grooves NG. Because hyaluronic acid is a viscous material, there is a limitation in filling the needle grooves NG with hyaluronic acid alone. However, the hyaluronic acid dissolved in the water has a low viscosity and a high fluidity, and thus may be filled in the needle grooves NG.

In an embodiment, the effective ingredient EM may be contained in the base material BM. The base material BM and the effective ingredient EM may be mixed with each other, and the effective ingredient EM may be injected into the needle grooves NG together with the base material BM being dissolved in the buffer solution BS.

At this time, the effective ingredient EM is not dissolved in the buffer solution BS. Therefore, the effective ingredient EM mixed in the base material being injected into the mold M is the same as the effective ingredient EM in the finally manufactured microneedle 320. Because the effective ingredient EM does not react with the buffer solution BS, the effectiveness of the effective ingredient EM does not change in the manufacturing process.

In another embodiment, the buffer solution BS may dissolve the effective ingredient EM. The effective ingredient EM is contained in the base material BM, and the buffer solution BS dissolves both the base material BM and the effective ingredient EM. For example, when the buffer solution BS is water and the effective ingredient EM is water-soluble, the effective ingredient EM may be dissolved in the buffer solution BS. Even when the effective ingredient EM is dissolved in the buffer solution BS, the effect of the effective ingredient EM is not changed.

In an alternative embodiment, the diffusion of the base material BM may be adjusted by controlling the environment.

For example, the diffusion rate of the base material BM may be increased by adjusting the temperature or humidity of a chamber in which the mold M is arranged.

As another example, an additive (not shown) for increasing the diffusion rate may be injected into the buffer solution BS to increase the diffusion rate of the base material BM.

As another example, the mold M may be mounted on a stirrer (not shown), and the diffusion rate of the base material BM may be increased by the vibration generated by the driving of the stirrer.

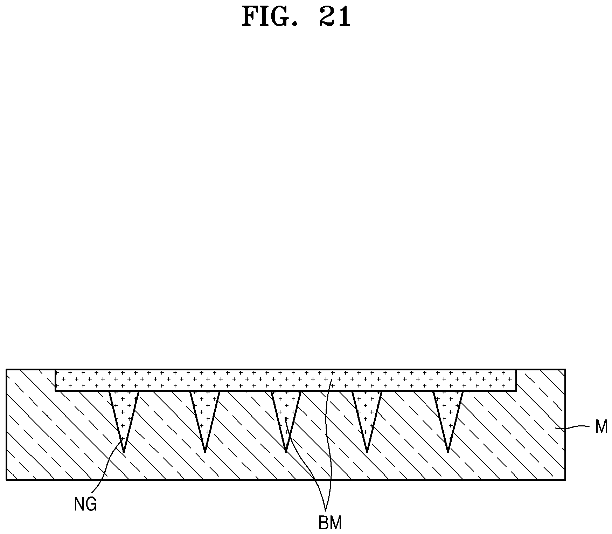

In the drying of the mold (S400), the microneedles 320 may be manufactured by drying the buffer solution BS (see FIG. 21).

The mold M is mounted on the drying module 30A, and then the drying module 30A is driven to dry the mold M. The buffer solution BS stored in the base mold M may be removed by the drying module 30A.

When the buffer solution BS is dried, the concentration of the base material BM is increased. Thereafter, the buffer solution BS is completely removed, the moisture included in the base material BM is also removed, and thus, the microneedles 320 formed of the base material BM are prepared.

Because the buffer solution BS is completely removed, only the base material BM may be effectively stored in the needle grooves NG. The base material BM changes to a solid, and the microneedle 320 has a certain stiffness.

The microneedle 320 formed as a solid has the effective ingredient EM therein. The effective ingredient EM remains included in the base material BM.

The base material BM suitable for forming the microneedles has a certain viscosity. Because the needle grooves NG of the mold M are significantly finely manufactured, it is difficult to completely fill the needle grooves NG with the base material BM having the certain viscosity.

When the base material BM is not completely filled in the needle groove NG, the tip of the microneedle is formed to be blunt. Because the microneedle 320 is required to be inserted through the skin of a patient, the microneedle 320 needs to have a sharpened tip. However, the base material BM having the certain viscosity is not completely filled in the needle groove NG, thus the tip of the microneedle 320 is formed to be blunt due to an unfilled space, and accordingly, it is difficult for the microneedle 320 to be attached to the skin of the patient and the drug delivery effect is reduced.

In addition, in the base material BM having the viscosity, gas such as air may be stored in the form of bubbles during a process of manufacturing the microneedle patch 300. Even when the base material BM is squeezed and injected into the needle grooves NG, the bubbles may still exist in the needle grooves NG. The gas remaining in the bubbles in the base material BM may seriously degrade the quality of the microneedle 320. Because the microneedle 320 is to be implanted into the skin of the patient, the microneedle 320 should not contain foreign substances including gas. When the gas in the bubbles is injected into the patient, the safety of the patient may be threatened.

By performing the method for manufacturing a microneedle patch according to the present disclosure, the mold is completely filled with the base material, and thus, a microneedle patch having a fine shape and improved quality may be manufactured. Because the base material is completely filled in the needle groove by the buffer solution, the microneedle is formed to have a sharpened tip, and thus may be easily attached to the skin of the patient. In addition, because foreign substances such as air are not injected into the needle grooves in the manufacturing process, a high-quality microneedle patch may be manufactured.

In detail, in order to manufacture a microneedle having a high stiffness, it is required to select high-molecular-weight 1.4 MDa 10 % HA as the base material. However, the high-molecular-weight 1.4 MDa 10 % HA has a relatively high viscosity, and thus is difficult to be completely injected into the mold. According to the method of manufacturing a microneedle patch of the present disclosure, when purified water, which is used as the buffer solution, is injected into the mold and the high-molecular-weight 1.4 MDa 10 % HA is dissolved in the buffer solution, the base material is completely injected into the needle grooves. Thereafter, when the purified water is dried, microneedles are manufactured in the shape of the needle grooves.

By using the method of manufacturing a microneedle patch according to the present disclosure, a microneedle patch having a complicated and fine needle shape may be manufactured. In the process of manufacturing the microneedle patch, as the base material is diffused into the buffer solution, the viscosity of the base material is decreased and the fluidity of the base material is increased. Although the shape of the needle groove is complicated and sophisticated, the fluidity of the base material may allow the base material to be completely filled in the needle groove.

Referring to FIG. 22, the microneedle patch 300, which is manufactured by using the apparatus 2 for manufacturing a microneedle patch or the method of manufacturing a microneedle patch, may include the base 310 and the microneedle 320 having a single-layer structure. The microneedle 320 may include the effective ingredient EM therein.

The base material BM and the effective ingredient EM of the microneedle patch 100 described above may be applied to the microneedle patch 300.

The microneedle 320 is formed by dissolving the base material BM in the buffer solution and then drying the buffer solution BS. At this time, the buffer solution BS may be completely removed.

In an embodiment, the buffer solution may include water, and the base material BM may include hyaluronic acid.

In an embodiment, the effective ingredient EM may be contained in the base material BM, and may not be dissolved in the buffer solution.

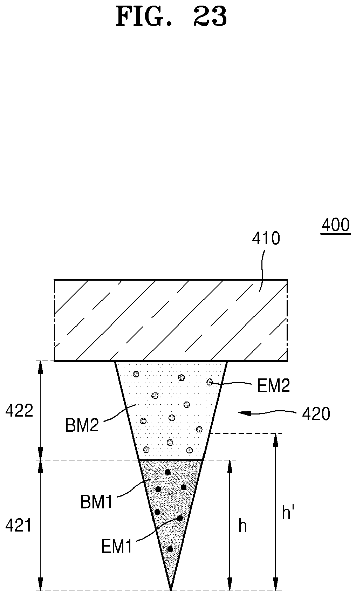

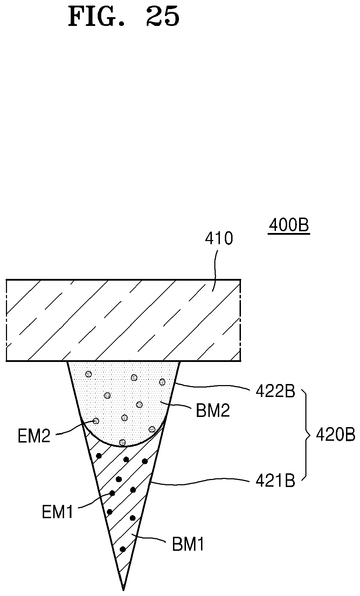

FIGS. 23 to 25 are diagrams illustrating other embodiments of the microneedle patch 300 of FIG. 22.

Referring to FIG. 23, a microneedle patch 400 may be manufactured by using the apparatus 2 for manufacturing a microneedle patch or the method of manufacturing a microneedle patch described above. The microneedle patch 400 may include a base 410 and a multi-layered microneedle 420.

The multi-layered microneedle patch 400 may be manufactured by driving the apparatus 2 for manufacturing a microneedle patch a plurality of times or by performing the method of manufacturing a microneedle patch a plurality of times.

In detail, a first buffer solution is injected into the needle groove NG. The first buffer solution is not completely filled in the needle groove NG.

For example, the first buffer solution may be filled with a volume greater than that of a first layer 421. The first buffer solution may be filled to a height h1', which is higher than a height h1 in FIG. 23.

A first base material BM1 is arranged in the mold M and is diffused into the first buffer solution. Because the first buffer solution dissolves the first base material BM1, the tip of the needle groove NG is filled with the first base material BM1.

Thereafter, the first buffer solution is removed by drying the mold M filled with the first buffer solution and the first base material BM1. Consequently, the first layer 421 is formed of the first base material BM1 in the tip of the needle groove NG. As the first buffer solution is removed in the drying process, the first layer 421 is formed to have the height h1.

Thereafter, a second buffer solution is injected into the needle groove NG. The second buffer solution is filled in the needle groove NG and onto the first layer 421.

A second base material BM2 is arranged in the mold M and is diffused into the second buffer solution. Because the second buffer solution dissolves the second base material BM2, the second base material BM2 is filled on the first layer 421.

Thereafter, the second buffer solution is removed by drying the mold M filled with the second buffer solution and the second base material BM2. Accordingly, a second layer 422 is formed of the second base material BM2 and on the first layer 421, and thus the microneedle 420 has a multi-layer structure.

At least one of the first layer 421 and the second layer 422 may include an effective ingredient. As illustrated in FIG. 23, the first effective ingredient EM1 may be included in the first layer 421, and the second effective ingredient EM2 may be included in the second layer 422. However, the present disclosure is not limited thereto, and only the first effective ingredient EM1 or only the second effective ingredient EM2 may be included. In addition, a plurality of effective ingredients may be mixed in each layer.

The first layer 421 may be formed to have a greater strength than that of the second layer 422. A process of drying the first layer 421 is additionally performed in a process of manufacturing the second layer 422. The strength of the first layer 421 may be enhanced by the additional drying process. As the strength of the first layer 421 is enhanced, the microneedle patch 400 may be easily attached to the skin of the patient.

In an embodiment, the second buffer solution may dissolve only the second base material BM2 and but not dissolve the first base material BM1. Even when the second buffer solution is injected into the needle groove NG after the first layer 421 is formed of the first base material BM1, the first layer 421 may not be dissolved by the second buffer solution. Consequently, the first layer 421 and the second layer 422 are clearly distinguished from each other, and thus, target positions of the first layer 421 and the second layer 422 may be clearly distinguished from each other.

In the microneedle patch 400, the microneedle 420 arranged on one surface of the base 410 has the multi-layer structure, and thus may accurately deliver the effective ingredient EM to a target point. Because the microneedle 420 includes a plurality of layers, the effective ingredient EM may be included in each layer. For example, the first effective ingredient EM1 may be included in the first layer 421, and the second effective ingredient EM2 may be included in the second layer 422. Consequently, in the microneedle patch 400, the depth at which each effective ingredient is to be activated may be adjusted according to the height of each layer. That is, the microneedle patch 400 may deliver the first and second effective ingredients EM 1 and EM2 to any one of an epidermis, a dermis, subcutaneous fat, and muscle, respectively.

Because the microneedle patch 400 has the multi-layer structure, the biodegradation rates of the layers may be set to be different from each other. In the microneedle 420, the decomposition rates of the first layer 421 and the second layer 422 may be set to be different from each other, and thus, the first effective ingredient EM1 and the second effective ingredient EM2 may be activated at different points of time.

Because the microneedle patch 400 has the multi-layer structure, and the strengths of the layers may be set to be different from each other. By setting the strength of the first layer 421 to be greater than the strength of the second layer 422, the microneedle 420 may be easily inserted into the skin.

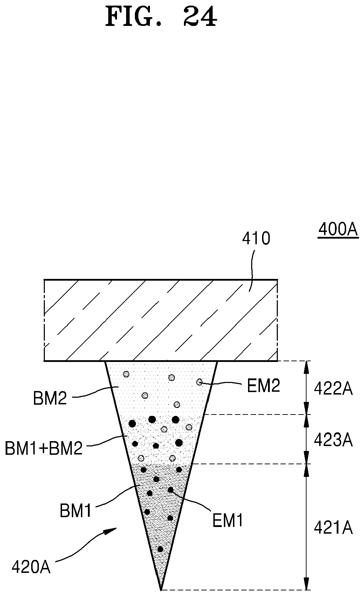

Referring to FIG. 24, a microneedle patch 400A may include the base 410 and a microneedle 420A.

The microneedle 420A may include a first layer 421A, a second layer 422A, and a transition layer 423A between the first layer 421A and the second layer 422A. The transition layer 423A may include a mixture of the first base material BM1 and the second base material BM2.

The second buffer solution may dissolve both the second base material BM2 and the first base material BM1. When the second buffer solution is injected into the needle groove NG after the first layer 421A is formed of the first base material BM1, a portion of the upper surface of the first layer 421A may be dissolved.

Thereafter, when the second base material BM2 is injected onto the upper surface of the first layer 421A, the transition layer 423A may be formed in the boundary region between the first layer 421A and the second layer 422A, and the first base material BM1 dissolved in the first layer 421A and the additionally injected second base material BM2 may be mixed with each other in the transition layer 423A.

The transition layer 423A may increase the coupling force between the first layer 421A and the second layer 422A, thereby increasing the strength of the microneedle 420A. The transition layer 423A may prevent the characteristics of the first layer 421A and the second layer 422A from being rapidly changed, and may increase the coupling force between the first layer 421A and the second layer 422A.

Referring to FIG. 23, a microneedle patch 400B may be manufactured by using the apparatus 2 for manufacturing a microneedle patch or the method of manufacturing a microneedle patch described above. The microneedle patch 400B may include a base 410B and a multi-layered microneedle 420B.

The microneedle 420B may include a first layer 421B and a second layer 422B, which constitute a layered structure.

First, the first base material BM1 is injected into the mold M filled with the first buffer solution. As the first buffer solution is removed during a drying process, the first layer 421B formed of the first base material BM1 may have a curved surface.

Thereafter, the second buffer solution is filled in the mold M, and the second base material BM2 is diffused into the second buffer solution. As the second buffer solution is removed during a drying process, the second layer 422B may be formed on the first layer 421B.

Although the present disclosure has been described with reference to the embodiments illustrated in the drawings, they are merely exemplary, and it will be understood by one of skill in the art that various modifications and equivalent embodiments may be made therefrom. Therefore, the true technical protection scope of the present disclosure should be determined by the appended claims.

The particular implementations shown and described herein are illustrative examples of embodiments and are not intended to otherwise limit the scope of embodiments in any way. Moreover, no item or component is essential to the practice of the present disclosure unless the item or component is specifically described as "essential" or "critical".

The term "the" and other demonstratives similar thereto in the descriptions of embodiments (especially in the following claims) should be understood to include a singular form and plural forms. Furthermore, recitation of ranges of values herein are merely intended to serve as a shorthand method of referring individually to each separate value falling within the range, unless otherwise indicated herein, and each separate value is incorporated into the specification as if it were individually recited herein. Finally, the operations of all methods described herein may be performed in any suitable order unless otherwise indicated herein or otherwise clearly contradicted by context. The present disclosure is not limited to the described order of the operations. The use of any and all examples, or exemplary language (e.g., "and the like") provided herein, is intended merely to better illuminate embodiments and does not pose a limitation on the scope of the embodiments unless otherwise claimed. In addition, various modifications, combinations, and adaptations will be readily apparent to those skilled in this art without departing from the following claims and equivalents thereof.

## Claims

1. A method of manufacturing a microneedle patch, the method comprising:
filling a mold having a plurality of needle grooves with a base material;
forming a subatmospheric pressure in the mold;
rotating the mold about a rotation axis; and
drying the base material filled in the needle grooves.

2. The method of claim 1, wherein, in the forming of the subatmospheric pressure in the mold, bubbles in the base material filled in the needle grooves are removed or gas between the base material and the needle grooves is removed.

3. The method of claim 1, wherein, in the rotating of the mold about the rotation axis, the base material is pushed in an axial direction of the needle grooves by centrifugal force.

4. The method of claim 1, further comprising, prior to the forming of the subatmospheric pressure in the mold, in advance rotating the mold filled with the base material about the rotation axis.

5. The method of claim 1, wherein the rotation axis is perpendicular to an axial direction of the needle grooves.

6. An apparatus for manufacturing a microneedle patch, the apparatus comprising:
a mold having a plurality of needle grooves into which a base material is injected;
a pressure module configured to create a subatmospheric pressure in the mold;
a rotation module configured to rotate the mold about a rotation axis; and
a drying module configured to dry the base material filled in the needle grooves.

7. The apparatus of claim 6, wherein, after bubbles in the base material are removed or gas between the base material and the needle grooves is removed by the pressure module, the base material is pushed in an axial direction of the needle grooves by centrifugal force generated by the rotation module being driven.

8. A method of manufacturing a microneedle patch, the method comprising:
filling a mold having a plurality of needle grooves with a buffer solution;
arranging a base material on the needle grooves;
diffusing the base material into the buffer solution; and
drying the mold.

9. The method of claim 8, wherein, in the diffusing of the base material into the buffer solution, the base material is injected into the needle grooves.

10. The method of claim 9, wherein the buffer solution dissolves the base material.

11. The method of claim 10, wherein the buffer solution comprises water and the base material comprises hyaluronic acid.

12. The method of claim 10, wherein an effective ingredient is included in the base material.

13. The method of claim 8, wherein, in the drying of the mold, the buffer solution is removed, and thus the base material is hardened in the needle grooves.
